# EUROPÄISCHE PATENTANMELDUNG

(11) **EP 1 069 183 A2**
(43) Veröffentlichungstag der Anmeldung: **17.01.2001**
(21) Anmeldenummer: 00111897.5
(22) Anmeldetag: 13.06.2000
(51) Int. Cl.: C12N 11/08, C12P 41/00, C12N 9/20

(54) **Immobilisierte Lipase**

(30) Priorität: 09.07.1999 DE 19931847
(71) Anmelder: BASF AKTIENGESELLSCHAFT, 67056 Ludwigshafen (DE)
(72) Erfinder: Friedrich, Thomas, Dr., 64283 Darmstadt (DE); Stürmer, Rainer, Dr., 67127 Rödersheim-Gronau (DE)

(57) **Zusammenfassung**

Die vorliegende Erfindung betrifft ein Verfahren zur Herstellung von immobilisierter Lipase indem man eine Rohlipaselösung mit Polyolefin-Partikel in Kontakt bringt. Ferner betrifft die Erfindung die immobilisierte Lipase selbst und ein Verfahren zur Herstellung optisch aktiver Verbindungen.

## Beschreibung

Die vorliegende Erfindung betrifft ein Verfahren zur Herstellung von immobilisierter Lipase, die immobilisierte Lipase selbst und ein Verfahren zur enzymkatalytischen Umwandlung in Gegenwart der immobilisierten Lipase.

Lipasen können in Lösung als enzymatische Katalysatoren zur Umwandlung von Substraten verwendet werden. Gegenüber den freien Lipasen zeichnen sich immobilisierte Lipasen durch eine erhöhte Stabilität und Standzeit unter kontinuierlicher und diskontinuierlicher Reaktionsführung sowie durch eine leichte Rückgewinnung der katalytisch aktiven Spezies bei diskontinuierlichen Reaktionsansätzen aus.

Es ist bekannt, Lipasen durch Adsorption an einen festen Träger zu immobilisieren. Ferner ist bekannt, immobilisierte Lipasen durch Inkontaktbringen von Polyolefinpartikel mit einer wäßrigen Lösung einer gereinigten Lipase herzustellen.

EP 232 933 beschreibt die Immobilisierung einer gereinigten Lipase aus wäßriger Lösung durch Adsorption an hydrophobe thermoplastische Polymere, wie beispielsweise aliphatische Polyolefine. Die immobilisierte Lipase wird zur Hydrolyse von Fett verwendet.

WO 90/15868 lehrt die Immobilisierung von gereinigter *Candida antarctica* Lipase aus wäßriger Lösung durch Adsorption an, mit organischen Lösungsmitteln vorbehandelte, aliphatische Polyolefine. Die immobilisierte Lipase wird zur Estersynthese verwendet.

In WO 94/28118 wird vor der Immobilisierung der gereinigten Lipase an hydrophobe Trägermaterialien eine nichtionische oberflächenaktive Substanz zugesetzt.

Alle Verfahren des Standes der Technik haben den Nachteil, daß die Lipase vor der Immobilisierung an den festen Träger von weiteren Proteinen, Enzymen und anderen Zellbestandteilen der Rohlipaselösung gereinigt wird. Dieser Reinigungsschritt durch Fällungs- und chromatographische Verfahren ist zeitaufwendig und teuer.

Ferner zeigen die nach dem Stand der Technik hergestellten immobilisierten Lipasen verglichen mit den freien Lipasen eine stärker verringerte Aktivität und müssen durch Zugabe von beispielsweise oberflächenaktiven Substanzen wieder aktiviert werden.

Die Zugabe von oberflächenaktiven Substanzen ist auch zur Aktivierung freier Lipasen in organischer Lösung nötig, die nach dem Stand der Technik aus einer Rohlipaselösung gereinigt wurden (WO 95/17504).

Weiterhin ist die Standzeit der nach dem Stand der Technik hergestellten immobilisierten Lipasen noch nicht optimal.

Der Erfindung lag daher die Aufgabe zugrunde, den geschilderten Mängeln abzuhelfen, ein neues vereinfachtes Verfahren zur Herstellung von immobilisierter Lipase und neue, durch ein vereinfachtes Verfahren hergestellte, immobilisierte Lipasen mit optimierten Eigenschaften, bereitzustellen.

Demgemäß wurde ein neues Verfahren zur Herstellung von immobilisierter Lipase gefunden, in dem man eine Rohlipaselösung mit Polyolefin-Partikel in Kontakt bringt.

Unter einer Rohlipaselösung wird beispielsweise eine Lipaselösung verstanden die mehr als 2 Gew.-%, vorzugsweise mehr als 5 Gew.-%, besonders bevorzugt mehr als 15 Gew.-% Verunreinigungen, wie beispielsweise weitere Proteine, andere zelluläre Bestandteile des Lipase-produzierenden Organismus oder Reste von Nährmedien enthält. Die Lipase kann in wäßriger Lösung oder auch in wäßrigen Puffersystemen oder in organischen Lösungsmitteln wie beispielsweise in gegebenenfalls halogenierten aliphatischen oder aromatischen Kohlenwasserstoffen wie beispielsweise Toluol vorliegen. Bevorzugt ist eine wäßrige Rohlipaselösung.

Bevorzugte wäßrige Rohlipaselösungen sind beispielsweise Kulturbrühen, die man durch Kultivieren eines Lipase-produzierenden Organismus in einem wäßrigem Nährmedium erhält oder die durch Dispergieren und/oder Homogenisieren eines Lipase-produzierenden Organismus oder eines Lipase-produzierenden Zellgewebes, wie beispielsweise eines tierischen Organs oder einer Pflanze, in einem wäßrigen, gegebenenfalls einen Puffer oder weitere Lipasestabilisierende Inhaltsstoffe enthaltenden Lösungsmittel erhältlich sind.

Vorzugsweise wird die Rohlipaselösung vor Inkontaktbringen mit den Polyolefin-Partikel von Zellen durch an sich bekannte Abtrennungsmethoden, wie Zentrifugieren oder Abfiltrieren gereinigt.

Das Inkontaktbringen erfolgt beispielsweise durch Eintragen der Polyolefinpartikel in die Rohlipaselösung.

Bei Inkontaktbringen der Rohlipaselösung mit den Polyolefinpartikeln wird die Lipase an die Polyolefinpartikel adsorbiert. Dabei war überraschend, daß Polyolefinpartikel eine sehr hohe Selektivität gegenüber Lipasen aufweisen, so daß nur die Lipase und gegebenenfalls deren Bruchstücke und nicht - oder nur in sehr geringem Umfang (in der Regel < 2 Gew.-%) - die weiteren Proteine aus der Rohlipaselösung an das Polyolefin adsorbiert werden.

Der Schritt der Adsorbtion stellt somit gleichzeitig einen Reinigungsschritt der Lipase von den weiteren Proteinen und Enzymen der Rohlipaselösung dar, so daß der Schritt einer weiteren Aufreinigung der Rohlipaselösung vor dem Immobilisieren auf dem festen Träger entfallen kann. Die auf diese Weise hergestellten immobilisierten Lipasen weisen, neben vereinfachten Herstellung, gegenüber den immobilisierten Lipasen des Standes der Technik folgende Vorteile auf:
- Die immobilisierte Lipase hat eine höhere Standzeit.
- Die Zugabe von aktivierenden Substanzen, wie beispielsweise Ölsäure ist nicht mehr nötig und bewirkt keine Aktivitätssteigerung.

Prinzipiell können für das erfindungsgemäße Verfahren auch weiter aufgereinigte Rohlipaselösungen verwendet werden. Eine Aufreinigung der Rohlipaselösung kann beispielsweise bis zu dem Punkt erfolgen, bei dem die Zugabe von Ölsäure zur immobilsierten Lipase wieder einen Aktivitätsschub bewirkt.

Als Aufreinigungsschritte kommen alle gängigen Verfahren zur Proteinreinigung, wie beispielsweise Ionenaustauschchromatographie, Molekularsiebchromatographie, hydrophobe Chromatographie und Fällungsmethoden in Frage.

Bevorzugt ist aber die Verwendung einer ungereinigten, zellfreien Rohlipaselösung im erfindungsgemäßen Verfahren.

Bevorzugt ist demnach ein Verfahren zur Herstellung von immobilisierter Lipase in dem man als Rohlipaselösung eine zellfreie Kulturbrühe verwendet, die durch
a) Kultivieren eines Lipase-produzierenden Organismus,
b) gegebenenfalls anschließendem Dispergieren und/oder Homogenisieren des Organismus in einer Lösung und
c) anschließendem Abtrennen der Zellen
erhältlich ist.

Unter einem Lipase-produzierenden Organismus wird ein Organismus verstanden, der auf natürlichem Wege oder durch gentechnische Veränderung, beispielsweise durch Insertion eines Lipasegens in das Genom des Organismus, in der Lage ist, eine Lipase zu produzieren. Unter Organismus werden Mikroorganismen, Pflanzen und Tiere sowie auch Zellgewebe tierischen oder pflanzlichen Ursprungs verstanden.

Bevorzugte bakterielle und pilzliche Lipasen stammen aus den Organismen der Gattung *Aspergillus*, *Arthrobacter*, *Alcaligenes*, *Bacillus*, *Brevibacterium*, *Pseudomonas*, *Burkholderia*, *Chromobacterium*, *Candida*, *Fusarium*, *Geotrichum*, *Humicola*, *Mucor*, *Pichia*, *Penicillium*, *Rhizomucor*, *Rhizopus oder Thermus.*

Besonders bevorzugte bakterielle und pilzilche Lipasen sind Lipasen aus den Gattungen und Arten Arthrobacter *sp., Alcaligenes sp., Aspergillus niger, Aspergillus oryzae, Bacillus cereus, Bacillus subtilis, Bacillus coagulans, Brevibacterium ammoniagenes, Burgholderi plantarii, Candida antarctica, Candida cylindracea, Candida lipolytica, Candida utilis, Candida rugosa, Chromobacterium viscosum, Fusarium solani, Geotrichum candidum, Humicola lanuginosa, Mucor sp., Mucor japonicus, Mucor javanicum, Mucor miehei, Pichia miso, Rhizopus nigricans, Rhizopus oryzae, Rhizopus arrhizus, Rhizopus sp., Rhizomucor miehei, Rhizopus arrhizus, Rhizopus delemar, Rhizopus niveus, Penicillin acylase, Penicillin roqueforti, Thermus aquaticus, Thermus flavus, Thermus thermophilus, Chromobacterium viscosum, Pseudomonas sp., Pseudomonas putida, Pseudomonas fluorescens, Pseudomonas cepacia, Pseudomonas burkholderia oder Pseudomonas aeruginosa.*

Bevorzugte tierische und pflanzliche Lipasen sind Schweinepankreaslipase (PPL) und Weizenkeimlipase.

Besonders bevorzugt ist die Lipase aus *Pseudomonas burkholderia* (ältere Bezeichnung: *Burgholderi plantarii*) oder *Pseudomonas aeruginosa* bzw. die Verwendung von *Pseudomonas burkholderia* oder *Pseudomonas aeruginosa* als Lipase-produzierenden Organismus.

Die Kultivierung von Mikroorganismen oder pflanzlichen oder tierischen Zellkulturen kann in an sich bekannter Weise, beispielsweise durch Fermentation in einem Nährmedium erfolgen, daß neben Nährstoffen, Spurenelementen und gegebenenfalls Antibiotika beispielsweise ein Puffersystem zur Stabilisierung der Proteine und Enzyme enthält. Der Schritt b) der Dispergierung und/oder Homogenisierung kann hier in der Regel entfallen.

Pflanzen, Tiere sowie Zellgewebe tierischen oder pflanzlichen Ursprungs wie Organe oder Pflanzenteile können in an sich bekannter Weise, beispielsweise in Nährböden bzw. in Tieren kultiviert, und in an sich bekannter Weise geerntet oder isoliert werden. Die Herstellung der Kulturbrühe erfolgt dann vorzugsweise, in an sich bekannter Weise durch Dispergieren und/oder Homogenisieren der Pflanzen oder Zellgewebe in einem Lösungsmittel, vorzugsweise in Wasser oder einer wäßrigen Pufferlösung und anschließender Abtrennung der Zellen.

Unter Polyolefinpartikel werden Partikel aus Polyolefinen verstanden. Bevorzugte Polyolefine sind Homopolymere oder Copolymere aus gegebenfalls substituierten Olefinen, wie beispielsweise Ethylen, Propylen, Butadien, Buten oder Octen. Besonders bevorzugte Polyolefinpartikel sind Partikel aus Polypropylen, wie beispielsweise Polypropylenpartikel die unter dem Namen ACCUREL^{(R)} (Fa. Akzo über Enka AG, Obernburg, Germany) erhältlich sind.

Die Teilchengröße und der Hohlraumanteil der Polyolefinpartikel ist nicht kritisch. Bevorzugte Partikel weisen aufgrund ihrer leichteren Handhabarkeit eine Teilchengröße von 100 µm bis 2000 µm, besonders bevorzugte Partikel eine Teilchengröße von 200 µm bis 1000 µm auf. Der Hohlraumanteil der Polyolefinpartikel beträgt vorteilhafterweise 40 % - 80 %, besonders bevorzugt 60 % - 70 %, ganz besonders bevorzugt 65 % auf.

Die Porengröße der Polyolefinpartikel beträgt vorzugsweise 0,01 µm bis 1 µm, besonders bevorzugt 0,05 µm bis 0,5 µm.

Die Beladung der Polyolefinpartikel mit Lipase ist nicht kritisch. Eine bevorzugte Beladung, bei der möglichst viel Lipase adsorbiert wird und nicht zuviel Lipase im Überschuß verloren geht ist von der Art des Polymers abhängig und kann über Routineversuche ermittelt werden. Bei der bevorzugten Verwendung von Polypropylenpartikel ist eine Beladung von 2 mg - 6 mg Lipase pro g Polyolefinpartikel bevorzugt, eine Beladung von 4,2 mg Lipase besonders bevorzugt.

Der Einfluß des pH-Wertes der Rohlipaselösung auf den Beladungsgrad ist nicht kritisch. Hohe Beladungen werden bei einem pH-Wert zwischen 4 und 7 erreicht. Bevorzugt ist ein pH-Wert zwischen 4,5 und 5,5, besonders bevorzugt ein pH-Wert von 4,8.

Der Einfluß der Ionenstärke der Rohlipaselösung auf den Beladungsgrad ist ebenfalls nicht kritisch. Hohe Beladungen werden bei einer Ionenstärke kleiner 500 mM erreicht. Besonders bevorzugt ist eine Ionenstärke kleiner 300 mM.

Die optimale Dauer des Beladungsverfahrens hängt von der Lipase und der Art der Polyolefinpartikel ab und kann durch Routineversuche bestimmt werden. In der Regel wird nach 4 bis 6 Stunden Kontaktzeit zwischen den Polyolefinpartikel und der Rohlipaselösung der Endwert der Beladung erreicht.

Die nach diesem Verfahren hergestellten immobilisierten Lipasen können direkt in den nachstehend beschriebenen enzymatischen Reaktionen eingesetzt werden. Eine Aktivierung, beispielsweise durch Zugabe von Ölsäure ist nicht nötig.

Vorteilhafterweise wird die immobilisierte Lipase vor dem Einsatz in einer Reaktion von nicht adsorbiertem Material gereinigt, beispielsweise durch eine Wäsche mit einem geeigneten Lösungsmittel, wie beispielsweise Wasser. Die immobilisierte Lipase kann anschließend gegebenenfalls nach an sich bekannten Methoden, wie beispielsweise durch Trocknung an Luft, getrocknet werden.

Die Erfindung betrifft ferner eine immobilisierte Lipase, erhältlich nach dem vorstehend beschrieben Herstellungsverfahren.

Die Erfindung betrifft weiterhin ein Verfahren zur enzymkatalytischen Umwandlung oder enantioselektiven Umwandlung von Substraten, indem man die Substrate in Gegenwart der erfindungsgemäßen immobilisierten Lipase umsetzt.

Die erfindungsgemäße immobilisierte Lipase wird demgemäß als Katalysator verwendet.

Als enzymkatalytische Umwandlungen werden chemische Reaktionen von Substraten verstanden, die die Lipasen im nicht immobilisierten, freien Zustand in Lösung katalysieren können. Beispielhaft seien folgende Reaktionen erwähnt:
Acylierung oder enantioselektive Acylierung von Alkoholen,
Acylierung oder enantioselektive Acylierung von Aminen,
Acylierung oder enantioselektive Acylierung von Axinoestern, wie beispielsweise Aminosäureester,
Verseifung (Hydrolyse) oder enantioselektive Verseifung (Hydrolyse) von Carbonsäureestern,
Acylierung oder enantioselektive Acylierung von Cyanhydrinen,
Verseifung oder enantioselektive Verseifung von Cyanhydrinestern,
Asymmetrisierung von Meso-diolen oder
Asymmetrisierung von Meso-diestern durch Verseifung.

Bevorzugte Verfahren sind Verfahren zur
Acylierung oder enantioselektive Acylierung von Alkoholen,
Acylierung oder enantioselektive Acylierung von Aminen,
Acylierung oder enantioselektive Acylierung von Aminoestern, wie beispielsweise Aminosäureester oder ein Verfahren zur
Verseifung (Hydrolyse) oder enantioselektiven Verseifung (Hydrolyse) von Carbonsäureestern.

Besonders bevorzugt wird bei diesen Verfahren immobilisierte Lipase aus *Pseudomonas burkholderia* (ältere Bezeichnung: *Burgholderi Plantarii*) oder aus Pseudomonas aeruginosa verwendet, die nach dem vorstehend beschriebenen erfindungsgemäßen Herstellungsverfahren hergestellt wurde.

Das Verfahren zur enzymicatalytischen Umwandlung oder enantioselektiven Umwandlung von Substraten ist dadurch gekennzeichnet, daß man die Substrate in Gegenwart der immobilisierten Lipase umsetzt.

Je nachdem ob es der Reaktionstyp erfordert, werden dabei vorzugsweise weitere Reagenzien zugegeben. So erfordert beispielsweise eine Acylierung die Zugabe eines Acylierungsmittels, während beispielsweise die Verseifung keine Zugabe weiterer Reagenzien benötigt.

Unter Substrat wird eine chemische Verbindung verstanden, die von Lipasen enzymkatalytisch umgesetzt, d.h. chemisch verändert werden kann. Bei enantioselektiven Umwandlungen sind Stereoisomerengemische, von denen nur ein Stereoisomeres umgesetzt wird, ebenfalls Substrate.

Beispielhaft seien Alkohole, Amine, Aminoester, Amide, Carbonsäureester, Thioester, Thiole, Cyanhydrine, Cyanhydrinester und Meso-diole und deren stereoisomerengemische als Substrate erwähnt. Bevorzugte Substrate sind Alkohole, Amine, Aminoester und Carbonsäureester bzw. racemische Alkohole, Amine, Aminoester und Carbonsäureester.

Das Verfahren wird vorzugsweise in Lösung, bei flüssigen Substraten mit oder ohne Lösungsmittel durchgeführt. Als Lösungsmittel können beispielsweise Wasser, organische Lösungsmittel oder auch wäßrig/organische Zweiphasengemische verwendet werden.

Vorzugsweise werden als organische Lösungsmittel Dioxan, THF, Diethylether, Methyl-t-butylether (MTBE), Toluol oder Heptan verwendet. Als wäßrig/organisches Zweiphasengemisch wird vorzugsweise ein Wasser/MTBE-Gemisch in beliebigem Verhältnis eingesetzt.

Bei Verfahrensdurchführung in Lösung ist die Substratkonzentration nicht kritisch, liegt aber vorzugsweise zwischen 0.5 Gew.-% und 50 Gew.-% bezogen auf die Lösung, besonders bevorzugt sind 20 bis 30 Gew.-%. Die Temperatur bei der Durchführung des Verfahrens ist ebenfalls nicht kritisch, ist aber nach oben durch die Temperaturstabilität der Lipase im Polymer beschränkt. Vorzugsweise wird das Verfahren bei 0°C bis 60°C durchgeführt, besonders bevorzugt sind 15°C bis 40°C.

Das Verfahren kann kontinuierlich oder diskontinuierlich durchgeführt werden. Für die kontinuierliche Verfahrensdurchführung leitet man beispielsweise in an sich bekannter Weise in einem Reaktor durch eine Schüttschicht aus immobilisierter Lipase eine flüssige mobile Phase. Die mobile Phase kann entweder eine Lösung von Substrat (und Reagenzien) bzw. die flüssigen Substrate (und Reagenzien) ohne Lösungsmittel sein. Die Durchflußrate ist nicht kritisch und richtet sich nach verfahrenstechnischen Gesichtspunkten wie Höhe, Durchmesser und Korngröße der Schüttschicht sowie nach der Ausgestaltung des Reaktors.

Als Reaktoren für das kontinuierliche Verfahren verwendet man vorzugsweise die für kontinuierliche, heterogenkatalytische Prozesse (Fluid/Feststoff-Reaktionen) üblichen Reaktoren (J. Hagen, Chemische Reaktionstechnik, VCH, Weinheim 1992, S. 165-169). Beispielhaft seien Wirbelschichtreaktoren und Festbettreaktoren, wie Rohrreaktor, Säulenreaktor, Vollraumreaktor, Hordenreaktor, Rohrbündelreaktor und Flachbett-Kontaktofen genannt.

In der diskontinuierlichen Verfahrensdurchführung werden die immobilisierten Lipasen in an sich bekannter Weise in einem Reaktor in einer Lösung von Substrat (und Reagenzien) bzw. in flüssigen Substraten (und Reagenzien) mit oder ohne Lösungsmittel suspendiert und die Suspension durchmischt. Als Reaktoren für das diskontinuierliche Verfahren verwendet man vorzugsweise die für diskontinuierliche, heterogenkatalytische Prozesse (Fluid/Feststoff-Reaktionen) üblichen Reaktoren mit Schüttel-, Misch- oder Rührvorrichtung. Beispielhaft sei der Rührkessel und sich davon ableitende Ausgestaltungen sowie Reaktionsgefäße mit Schüttelvorrichtung erwähnt.

Nach Beendigung der Reaktion (Erreichung des thermodynamischen Gleichgewichts) wird die immobilisierte Lipase, beispielsweise durch Dekantieren, Abzentrifugieren oder Abfiltrieren und Waschen isoliert und in weiteren Reaktionen verwendet.

In einer bevorzugten Ausführungsform des Verfahrens werden Substrate, die acylierbare funktionelle Gruppen wie z.B. Hydroxyl- oder Aminogruppen enthalten, wie Alkohole, Amine oder Aminosäureester in Gegenwart der immobilisierten Lipase als Katalysator und eines Acylierungsmittels acyliert oder enantioselektiv acyliert.

Diese enzymkatalytische Umwandlung wird bevorzugt in einem organischen Lösungsmittel wie beispielsweise Dioxan, THF, Diethylether, Methyl-t-butylether (MTBE), Toluol oder Heptan durchgeführt.

Besonders bevorzugt ist ein Verfahren zur Acylierung oder enantioselektiven Acylierung von Alkoholen, Aminen oder Aminosäureester oder racemischen Alkoholen, Aminen oder Aminosäureester in Gegenwart eines Acylierungsmittels und einer immobilsierten Lipase aus *Pseudomonas burkholderia* oder Pseudomonas aeruginosa.

Hinsichtlich der Alkohole, Amine und Aminosäureester gibt es praktisch keine Beschränkung. So können ein- und mehrwertige Alkohole, wie beispielsweise
1-Phenylethanol,
2-Chlor-1-phenylethanol,
2-Chlor-1-(m-chlorphenyl)-ethanol,
Pent-3-in-2-ol,
1-Butin-3-ol,
2-Hydroxy-4-phenylbuttersäureester,
a-Methyl-(1,3)-benzodioxol-5-ethanol,
2-propanol-1-(1,3-benzodioxol-4-yl),
trans-2-Methoxy-cyclohexanol oder
2-Methoxy-2-phenyl-ethanol oder deren Stereoisomerengemische,
ein und mehrwertige Amine oder deren Stereoisomerengemische oder
α, β oder γ-Aminosäureester wie beispielsweise die gegebenfalls mit Halogen substituierten C₁-C₄-Alkyl-, Alkylaryl-, Aryl-, C₂-C₆-Alkenyl- oder C₂-C₆-Alkinyl-Ester der natürlichen Aminosäuren oder deren Stereoisomerengemische
verwendet werden.

Als Acylierungsmittel werden organische Verbindungen verstanden, die in Gegenwart von Lipasen in Lösung als Acylüberträger fungieren können. Beispielhaft seien erwähnt:
Aliphatische, araliphatische oder aromatische Carbonsäuren die gegebenenfalls mit Halogen, wie Cl, Br, I, F substituiert sind (Acylierung), wie
C₁-C₆-Alkancarbonsäuren, z.B. Ameisensäure, Essigsäure, Propionsäure, Buttersäure oder
wie araliphatische oder aromatische Carbonsäuren z.B. Benzoesäure, 3-Phenylpropionsäure oder
die entsprechenden Carbonsäureester (Umesterung) wie beispielsweise
3-Phenylpropionsäureester oder Essigsäurealkylester, wie z.B. Essigsäureethylester.

Bevorzugte Carbonsäureester als Acylierungsmittel sind Vinylester der Formel I in der 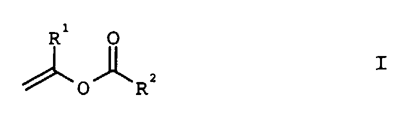
- R¹: Wasserstoff oder eine C₁-C₄-Alkyl-, vorzugsweise Methylgruppe und
- R²: Wasserstoff, C₁-C₁₈-Alkyl, welches gegebenenfalls mit Halogen substituiert ist, Phenyl oder (C₁-C₃-)Alkoxy-(C₁-C₄)-Alkyl bedeutet,
wie Vinylformiat, Vinylacetat, Vinylpropionat, Vinylbutyrat oder Vinyllaurat.

Acylierungsmittel sind weiterhin aliphatische, cycloaliphatische, araliphatische oder aromatische Carbonsäureanhydride und gemischte Carbonsäureanhydride (Acylierung) wie Essigsäureanhydrid, Bernsteinsäureanhydrid (BSA), Buttersäureanhydrid, 2-Ethylhexansäureanhydrid oder Methyl-Bernsteinsäureanhydrid. Im Falle der Verwendung von Bernsteinsäureanhydrid (BSA) oder anderer schlecht löslicher Anhydride als Acylierungsmittel kann Propylencarbonat besonders vorteilhaft zugemischt werden, um das BSA in Lösung zu bringen. Dies ist vor allem im Hinblick auf ein kontinuierliches Verfahren von Bedeutung.

In einer weiteren bevorzugten Ausführungsform des Verfahrens werden Carbonsäureester in Gegenwart der immobilisierten Lipase verseift oder enantioselektiv verseift.

In diesem Fall müssen keine weiteren Reagenzien zugegeben werden, dennoch ist die Anwesenheit von Wasser nötig. Bevorzugt wir die Verseifung von Carbonsäureester durch Zusatz von Wasser unter Verwendung eines vorzugsweise zweiphasigen Systems wie z.B. Wasser/MTBE in Gegenwart der immobilisierten Lipase durchgeführt.

Besonders bevorzugt ist ein Verfahren zur Verseifung oder enantioselektiven Verseifung von Carbonsäureestern in Gegenwart einer erfindungsgemäßen immobilsierten Lipase aus *Pseudomonas burkholderia* oder Pseudomonas aeruginosa.

Hinsichtlich der Carbonsäureester gibt es praktisch keine Beschränkung. So können beispielsweise

Verbindungen der Formel II oder deren Stereoisomerengemische 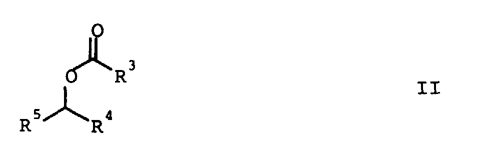 wobei
R³, R⁴ und R⁵ unabhängig voneinander Wasserstoff,
Halogen, wie beispielsweise F, Cl, Br oder I,
einen verzweigten oder unverzweigten, gegebenenfalls substituierten
C₁-C₈-Alkylrest wie beispielsweise gegebenenfalls substituiertes Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 1,2-Dimethylpropyl, 1,1-Dimethylpropyl, 2,2-Dimethylpropyl, 1-Ethylpropyl, Hexyl, 1-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl 1-Ethyl-2-methylpropyl, Heptyl oder Octyl,
C₂-C₆-Alkenylrest, wie beispielsweise gegebenenfalls substituiertes 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-entenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl oder 1-Ethyl-2-methyl-2-propenyl,
C₃-C₆-Alkinylrest wie beispielsweise gegebenenfalls substituiertes 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl oder 1-Ethyl-1-methyl-2-propinyl
oder C₃-C₈-Cycloalkylrest, wie beispielsweise gegebenenfalls substituiertes Cyclopropyl, Cyclobutyl, Cyclopentyl, Cyclohexyl und Cycloheptyl, Cyclooctyl,
einen gegebenenfalls substituierten
Arylrest, wie beispielsweise gegebenenfalls substituiertes Phenyl, 1-Naphtyl oder 2-Naphtyl,
Arylalkylrest, wie beispielsweise gegebenenfalls substituiertes Benzyl,
Heteroarylrest, wie beispielsweise gegebenenfalls substituiertes 2-Pyridyl, 3-Pyridyl, 4-Pyridyl, 2-Furyl, 3-Furyl, 2-Pyrrolyl, 3-Pyrrolyl, 2-Thienyl, 3-Thienyl, 2-Thiazolyl, 4-Thiazolyl, 5-Thiazolyl, 2-Oxazolyl, 4-Oxazolyl, 5-Oxazolyl, 2-Pyrimidyl, 4-Pyrimidyl, 5-Pyrimidyl, 6-Pyrimidyl, 3-Pyrazolyl, 4-Pyrazolyl, 5-Pyrazolyl, 3-Isothiazolyl, 4-Isothiazolyl, 5-Isothiazolyl, 2-Imidazolyl, 4-Imidazolyl, 5-Imidazolyl, 3-Pyridazinyl, 4-Pyridazinyl, 5-Pyridazinyl oder 6-Pyridazinyl, vorzugsweise 2-Pyridyl, 3-Pyridyl, 4-Pyridyl, 2-Furyl, 3-Furyl, 2-Thienyl, 3-Thienyl, 2-Thiazolyl, 4-Thiazolyl oder 5-Thiazolyl,
oder Heterocycloalkyl- oder -alkenylrest bedeuten.

Als ein bis dreifache Substituenten der C₁-C₈-Alkyl-, C₂-C₆-Alkenyl-, C₂-C₆-Alkinyl-, C₃-C₈-Cycloalkyl-, Aryl-, Arylalkyl-, Heteroaryl-, Heterocycloalkyl- oder -alkenylreste kommen beispielsweise Halogen-, Nitro-, Amino-, Hydroxy- oder Cyanogruppen, C₁-C₄-Alkyl-, C₁-C₄-Halogenalkyl-, C₁-C₄-Alkoxy-, C₁-C₄-Halogenalkoxy-, C₁-C₄-Alkylthio-, Hetaryl-, Arylreste oder der Rest -O-CO-C₁-C₄-Alkyl
in Frage.

Bevorzugte Carbonsäureester sind beispielsweise
1-Butin-3-ol-acetat,
1-Butin-3-ol-butyrat,
Essigsäure-1-phenyl-ethylester oder
2-Acetoxy-4-phenylbuttersäureester.

Das Verfahren zur enantioselektiven enzymkatalytischen Umwandlung von Substraten mit den erfindungsgemäßen immobilisierten Lipasen kann zur Abtrennung von Stereoisomeren und insbesondere zur Abtrennung von Enantiomeren oder Diastereomeren aus einem Stereoisomerengemisch des Substrats dienen. Besonders bevorzugt dient es zur Abtrennung von Enantiomeren oder Diastereomeren aus racemischen Substraten und damit zur Herstellung von optisch aktiven Verbindungen aus den jeweiligen raceinischen Gemischen.

Durch die enantioselektive Substratspezifität der immobilisierten Lipase, wird beispielsweise nur ein Enantiomeres des racemischen Substrats umgewandelt, das andere Enantiomere reagiert nicht. Die entstehenden Produkte lassen sich durch chemische, physikalische und mechanische Trennmethoden in an sich bekannter Weise leicht trennen. Beispielhaft seien Kristallisation, Fällung, Extraktion in zweiphasigen Lösungsmittelsystemen, chromatographische Trennverfahren, wie HPLC, GC oder Säulenchromatographie über Kieselgel oder thermische Trennungsverfahren wie Destillation genannt.

Demgemäß betrifft die vorliegende Erfindung weiterhin ein Verfahren zur Herstellung optisch aktiver Verbindungen, dadurch gekennzeichnet, daß man Stereoisomerengemische oder Racemate von Subtraten, die sich enzymkatalytisch von Lipasen umsetzen lassen, enantioselektiv in Gegenwart der erfindungsgenmäßen immobilisierten Lipase umsetzt und anschließend die Gemische auftrennt.

Mit dem erfindungsgemäßen Verfahren lassen sich vorzugsweise die optisch aktiven Verbindungen herstellen, die als Stereoisomerengemisch als Substrate von Lipasen umgesetzt werden können, bzw. von deren Stereoisomerengemisch mindestens ein Stereoisomer als Substrat von Lipasen umgesetzt werden kann.

Ein Verfahren zur enantioselektiven Acylierung von Alkoholen, Aminen oder Aminosäureester dient vorzugsweise zur Trennung von racemischen Alkoholen, Aminen bzw. Aminosäureester und damit zur Herstellung von otisch aktiven Alkoholen, Aminen bzw. Aminosäureester.

Ein Verfahren zur enantioselektiven Hydrolyse oder Verseifung von Carbonsäureestern dient vorzugsweise zur Trennung von racemischen Carbonsäureestern und damit zur Herstellung von otisch aktiven Carbonsäureestern.

Die nachstehenden Beispiele erläutern die Erfindung:

### Beispiel 1

### Herstellung immobilisierter Lipasen

### 1.1 Kultivieren eines Lipase-produzierenden Organismus am Beispiel der Ferinentation von Pseudomonas burkholderia

Der Stamm *Pseudomonas burkholderia* wurde in einer 14 l Fermentation eingesetzt. Die Vorkultur enthielt:
- 1: g/l MgSO₄ * 7 H₂0
- 3,5: g/l KH₂PO4
- 3,5: g/l K₂HPO4
- 5,0: g/l (NH₄)H₂PO₄
- 0,02: g/l CaCl₂ * 2 H₂O
- 10: g/l Yeast-Extrakt (Difco)

Jeweils 200 ml Medium wurden in einem 1 l Schüttelkolben mit 2 Bodenschikanen für 30 min bei 121 °C sterilisiert und in jeden Kolben 1 ml Trace-Elements (2-fach konzentriert) zugeben. Für einen 14 l Fermenter wurden 2 Schüttelkolben mit *Pseudomonas burkholderia* 7 - 9 Stunden bei 30 °C und 200 UPM inkubiert.

Fermenterkonfiguration:
Standardkonfiguration Techfors-Fermenter mit einer
pH-Elektrode (Säure- und Lauge-Regelung),
pO₂-Elektrode und einer
Antischaumelektrode (Russell).

Peripherie:
- 2 l Glasflasche mit Masterflex-Schlauch und einer Anstechnadel wurde mit ca. 2 kg Sojaöl gefüllt und 30 min bei 121°C sterilisiert.
- 2 leere 1 l Glasflaschen (für Säure und Lauge-Dosierung) mit Aristechnadeln wurden 30 min bei 121 °C sterilisiert. Anschließend wurden 500 ml Ammoniakwasser (25 %) und 500 ml Schwefelsäure (20 %) in die sterilisierten Flaschen gefüllt.
- 1 Glasflasche, gefüllt mit Tegosipon 3062 (ca. 500 ml) mit Anstechnadel wurde 30 min bei 121 °C sterilisiert.
- Sartorius-Dosierstrecke mit Waage und Watson Marlow-Pumpe zur Öldosierung für die Sojaölzugabe.

Der Mediumansatz für die Fermentation bestand aus:
- 110: g Yeast-Extract (Difco)
- 38,5: g K₂HPO₄
- 38,5: g KH₂PO4
- 55: g (NH₄)H₂PO₄
- 11: g MgSO₄ * 7 H₂0
- 0,22: g CaCl₂ * 2 H₂O
auf 11 l mit (VE-Wasser) auffüllen.

Der Ansatz wurde 30 min bei 121°C sterilisiert und anschließend 55 ml Trace-Elements (2-fach konzentriert) zugegeben.

Dieses Medium wurde mit 400 ml Vorkultur von *Pseudomonas burkholderi* beimpft und die Ferinentation 60 h unter den folgenden Bedingungen in einem 14 l-Fermenter durchgeführt:
- Temperatur:: 30°C
- Drehzahl:: 1000 UPM
- Begasung:: 11 l/min
- Druck:: 0,1 bar
- pH-Wert:: 6,5

Während der Fermentation wurde das Sojaöl zudosiert, wobei die Dosiermenge exponentiell mit der Zeit zunahm. Nach Beendigung der Fermentation wurde der Fermenter auf 15 °C abgekühlt und abgelassen.

Die Lipaseaktivität der abgelassenen Rohlipaselösung von Pseudomonas burkholderi betrug 8900 U/ml.

### 1.2 Immobilisierung

Vor der Immobilisierung wurden die aus den Fermentern abgelassene Rohlipaselösung von *Pseudomonas burkholderia* aus Beispiel 1.1. bei 5000 - 6000 rpm in der swing-out Zentrifuge RC-3B in 1 l Gefäßen abzentrifugiert.

Nach Abzentrifugieren der aus dem Fermenter abgelassenen Rohlipaselösung von Pseudomonas burkholderia betrug die Aktivität der trüben, zelifreien Rohlipaselösung 10500 U/ml.
8,3 mg Lipase entsprechen 250 000 U

Der zellfreie, trübe Überstand wurde in verschiedenen Konzentrationen (Verdünnung) bei unterschiedlichen Kontaktzeiten und unter verschiedenen Bedingungen (pH-Wert, Ionenstärke) mit Accurel^{(R)} 1004 (Polypropylenpartikel < 400 µm) und Accurel^{(R)} 1001 (Polypropylenpartikel 400 µm bis 1000 µm) inkubiert.

### 1.2.1 Immobilisierung auf Accurel^{(R)} 1004

Tabelle 1.1 stellt die Abhängigkeit der Adsorption (Prozentuale Beladung des Trägers verglichen mit der im Überstand verbliebenen freien Lipase) und der Aktivität der immobilisierten Lipase von der Lipasekonzentration (Beladung (mg Lipase/ g Träger) in der Rohlipaselösung dar.
- Kontaktzeit:: 2 h
- pH-Wert:: 7
- Ionenstärke:: 3,2 mS

**Tabelle 1.1**

| Beispiel | Beladung [mg Lipase/g Träger] | Beladung Lipase an Träger in [%] | Aktivität [U/g Träger] |
|---|---|---|---|
| 1.2.1 a) | 0,5 | 90 | 30 |
| 1.2.1 b) | 1 | 90 | 65 |
| 1.2.1 c) | 2,1 | 86 | 145 |
| 1.2.1 d) | 4,2 | 78 | 250 |
| 1.2.1 e) | 8,3 | 73 | 407 |

### 1.2.2 Immobilisierung auf Accurel^{(R)} 1001

Tabelle 1.2 stellt die Abhängigkeit der Adsorption (Prozentuale Beladung des Trägers verglichen mit der im Überstand verbliebenen freien Lipase) und der Aktivität der immobilisierten Lipase von der Lipasekonzentration (Beladung (mg Lipase/ g Träger) in der Rohlipaselösung dar.
- Kontaktzeit:: 2 h
- pH- Wert:: 7
- Ionenstärke:: 3,2 mS

**Tabelle 1.2**

| Beispiel | Beladung [mg Lipase/g Träger] | Beladung Lipase an Träger in [%] | Aktivität [U/g Träger] |
|---|---|---|---|
| 1.2.2 a) | 0,5 | 80 | 20 |
| 1.2.2 b) | 1 | 80 | 45 |
| 1.2.2 c) | 2,1 | 80 | 87 |
| 1.2.2 d) | 4,2 | 78 | 220 |
| 1.2.2 e) | 8.3 | 67 | 410 |

Bei allen weiteren Versuchen mit immobilisierter Lipase aus *Pseudomonas burkholderia* wurden 4,2 mg Lipase/g Träger eingesetzt und Accurel(R) 1001 als Polyolefinpartikel verwendet.

Tabelle 1.3 zeigt die Abhängigkeit der Adsorption (Prozentuale Beladung des Trägers verglichen mit der im Überstand verbliebenen freien Lipase) vom pH Wert.
- Kontaktzeit:: 1 h
- Ionenstärke:: 3.2 mS

**Tabelle 1.3**

| Beispiel | pH-Wert | Beladung Lipase an Träger in [%] |
|---|---|---|
| 1.2.2 f) | 4,8 | 81 |
| 1.2.2 g) | 5,8 | 73 |
| 1.2.2 h) | 6,8 | 71 |
| 1.2.2 i) | 7,8 | 68 |
| 1.2.2 j) | 8,8 | 62 |

Tabelle 1.4 zeigt die Abhängigkeit der Adsorption (Prozentuale Beladung des Trägers verglichen mit der im Überstand verbliebenen freien Lipase) von der Ionenstärke (Konzentration Na-Sulfat).
- Kontaktzeit:: 1 h
- pH-Wert:: 7

**Tabelle 1.4**

| Beispiel | Konzentration Na₂SO₄ in [M] | Beladung Lipase an Träger in [%] |
|---|---|---|
| 1.2.2 k) | 0 | 68 |
| 1.2.2 l) | 0,1 | 68 |
| 1.2.2 m) | 0,5 | 50 |
| 1.2.2 n) | 1 | 50 |
| 1.2.2 o) | 2 | 31 |

Tabelle 1.5 zeigt die Abhängigkeit der Adsorption (Prozentuale Beladung des Trägers verglichen mit der im Überstand verbliebenen freien Lipase) von der Kontaktzeit.
- pH-Wert:: 7
- Ionenstärke:: 3,2 mS

**Tabelle 1.5**

| Beispiel | Kontaktzeit in [h] | Beladung Lipase an Träger in [%] |
|---|---|---|
| 1.2.2 p) | 0,16 | 60 |
| 1.2.2 q) | 0,33 | 66 |
| 1.2.2 r) | 0,66 | 64 |
| 1.2.2 s) | 1 | 68 |
| 1.2.2 t) | 1,5 | 63 |
| 1.2.2 u) | 2,0 | 63 |
| 1.2.2 v) | 4,0 | 86 |
| 1.2.2 w) | 6,0 | 91 |
| 1.2.2 x) | 8,0 | 91 |
| 1.2.2 y) | 24 | 93 |

### 1.2.3 Desorption der Lipase vom Trägermaterial

### 1.2.3.1 Lipase aus Pseudomonas burgholderia

Die nach Beispiel 1.2.2 d) aus einer Rohlipaselösung von *Pseudomonas burgholderia* (Spur 1) hergestellte immobilisierte Lipase wurde abfiltriert (Filtrat der Rohlipaselösung = nichtadsorbierte Bestandteile der Rohlipaselösung = Spur 3), gewaschen (Spur 4) und mit einer desorbierenden SDS-Pufferlösung 5 min bei 95°C in Kontakt gebracht. Die Pufferlösung (Spur 2) enthält gelöst die wieder desorbierte und von den übrigen Bestandteilen der Rohlipaselösung (Spur 3) gereinigte Lipase.

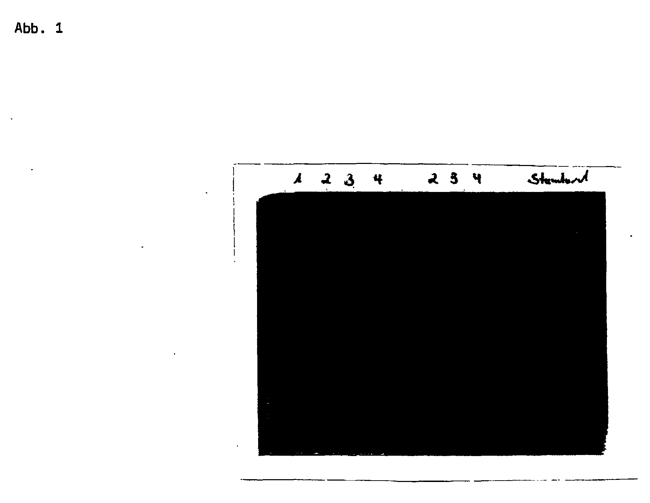

Abbildung 1 zeigt die entsprechende gel-elektrophoretische Autrennung der Spuren 1 bis 4.
Standard = Molekulargewichtsstandards: 207 kDa; 123 kDa; 86 kDa; 44,6 kDa; 31,4 kDa; 18,7 kDa; 7,2 kDa.

### 1.2.3.1 Lipase aus Pseudomonas aeruginosa

Die Herstellung der immobilisierten Lipase aus *Pseudomonas aeruginosa* erfolgte analog zu Beispiel 1.1 und Beispiel 1.2.2 d) unter Verwendung von *Pseudomonas aeruginosa* an Stelle von *Pseudomonas burkholderia*.

Die aus der Rohlipaselösung von *Pseudomonas aeruginosa* (Spur 1) hergestellte immobilisierte Lipase wurde abfiltriert (Filtrat der Rohlipaselösung = nichtadsorbierte Bestandteile der Rohlipaselösung = Spur 3) gewaschen (Spur 4) und mit einer desorbierenden SDS-Pufferlösung 5 min bei 95 °C in Kontakt gebracht. Die Pufferlösung (Spur 2) enthält gelöst die wieder desorbierte und von den übrigen Bestandteilen der Rohlipaselösung (Spur 3) gereinigte Lipase.

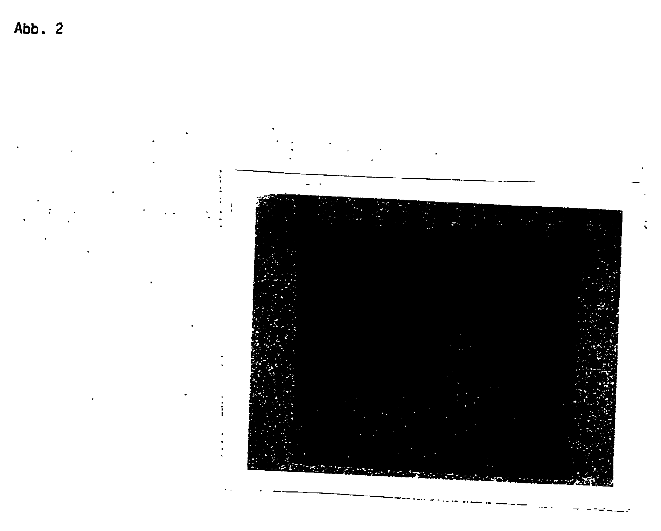

Abbildung 2 zeigt die entsprechende gel-elektrophoretische Auftrennung der Spuren 1 bis 4.
Standard = Molekulargewichtsstandards: 207 kDa; 123 kDa; 86 kDa; 44,6 kDa; 31,4 kDa; 18,7 kDa; 7,2 kDa.

### Beispiel 2

Enantioselektive Acylierung von racemischen 1-Phenyl-ethanol als Substrat mit Vinylpropionat als Acylierungsmittel in Gegenwart der nach Beispiel 1.2.2. d) hergestellten immobilisierten Lipase

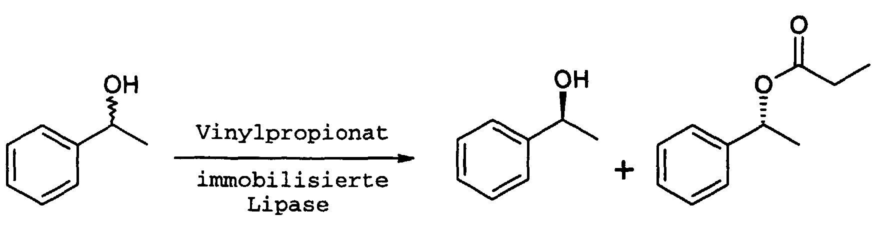

### Beispiel 2.1

### Diskontinuierliches Verfahren

Zunächst wurde eine Stammlösung aus 500 g (4.1 mol) 1-Phenylethanol und 246 g ( 2.46 mol) Vinylpropionat in 2 l MTBE bereitet. 5 ml dieser Lösung wurden zu jeweils einem 10 mg freier Lipase entsprechenden Aliquot der immobilisierten Lipase gegeben. Es wurde 12 h bei RT geschüttelt, von der immobilisierten Lipase abfiltriert und der Umsatz und die Enantioselektivität des acylierten Antipoden per GC bestimmt und in Tabelle 3 aufgelistet. Die Filterrückstände wurden mit MTBE gewaschen und erneut eingesetzt, um die Standzeit zu bestimmen. Die Versuchsdurchführung wurde bis zu 10 mal wiederholt. Als immobilisierte Lipasen wurden die in Beispiel 1.2.2 d) hergestellte immobilisierte Lipase aus *Pseudomonas burkholderi* verwendet. Zum Vergleich wurde der analoge Versuch mit der freien Lipase durchgeführt.

**Tabelle 3.1**

| | | |
|---|---|---|
| Umsatz (U in [%]) und Enantioselektivität (ee in [%]) des acylierten Antipoden bei der enantioselektiven Acylierung von racemischen 1-Phenyl-ethanol in Gegenwart der freien Lipase in einem diskontinuierlichen Verfahren. Das Verfahren wurde mit der aus dem vorhergehenden Versuch isolierten Lipase bis zu 10 mal wiederholt. | | |

| freie Lipase | | |
|---|---|---|
| Lauf | U | ee |
| 1 | 50.6 | 98 |
| 2 | 44.8 | 99 |
| 3 | 42.4 | 99 |
| 4 | 38.4 | 99 |
| 5 | 23.7 | 99 |
| 10 | 11.3 | 99 |

**Tabelle 3.2**

| | | |
|---|---|---|
| Umsatz (U in [%]) und Enantioselektivität (ee in [%]) des acylierten Antipoden bei der enantioselektiven Acylierung von racemischen 1-Phenyl-ethanol in Gegenwart der jeweiligen immobilisierten Lipase in einem diskontinuierlichen Verfahren. Das Verfahren wurde mit den aus dem vorhergehenden Versuch isolierten immobilisierten Lipase bis zu 10 mal wiederholt. | | |

| immobilisierte Lipase Pseudomonas burkholderi (Bsp. 1.2.2 d) | | |
|---|---|---|
| Lauf | U | ee |
| 1 | 50,0 | >99 |
| 2 | 49,9 | >99 |
| 3 | 49,9 | >99 |
| 4 | 49,8 | >99 |
| 5 | 49,9 | >99 |
| 10 | 49,7 | >99 |

### Beispiel 3.2

### Kontinuierliches Verfahren

In eine Chromatographiesäule (1,8 * 15 cm) wurde die immobilisierte Lipase aus Beispiel 1.2.2.f) eingefüllt, und es wurde mittels einer Pumpe ein auf 30-35 ml/h eingestellter Strom der Eduktlösung über das Immobilisat gepumpt. In regelmäßigen Abständen wurden Proben genommen, per GC der Umsatz und die Enantioselektivität bestimmt und in Tabelle 3.4 aufgelistet. Zum Vergleich wurde ein Lyophilisat der freien Lipase eingesetzt.

**Tabelle 3.4**

| | | | | | | | | | | | | |
|---|---|---|---|---|---|---|---|---|---|---|---|---|
| Umsatz (U) und Enantioselektivität (ee) in [%], gemessen in regelmäßigen Zeitabständen bei einem kontinuierlichen Verfahren der enantioselektiven Acylierung von racemischen 1-Phenyl-ethanol. Die Werte der immobilisierte Lipase aus Beispiel 1.2.2 d) werden mit den Werten der freien Lipase verglichen. | | | | | | | | | | | | |

| Zeit | 10 h | | 20 h | | 50 h | | 100 h | | 150 h | | 200 h | |
|---|---|---|---|---|---|---|---|---|---|---|---|---|
| | U | ee | U | ee | U | ee | U | ee | U | ee | U | ee |
| freie Lipase | 50,1 | 99 | 48,2 | 99 | 32,1 | 99 | 15,4 | 99 | - | | - | |
| Imm. Lipase Beisp.1.2.2 d) | 50,3 | 99 | 50,0 | 99 | 50,1 | 99 | 49,9 | 99 | 49,8 | 99 | 49,9 | 99 |

### Beispiel 4

### Enantioselektive Acylierung von racemischen trans-2-Methoxycyclohexanol als Substrat mit Bernsteinsäureanhydrid als Acylierungsmittel in Gegenwart der nach Beispiel 1.2.2 d) hergestellten immobilisierten Lipase

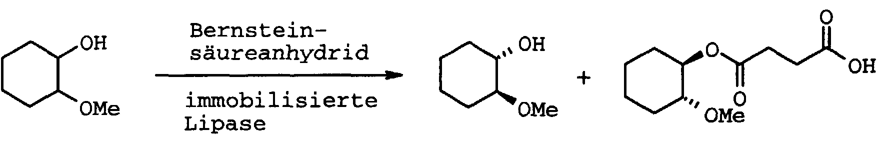

### Beispiel 4.1

### Diskontinuierliches Verfahren

Zunächst wurde eine Stammlösung aus 1100 g (8,46 mol) trans-2-Methoxycyclohexanol und 470 g (4,65 mol) Bernsteinsäureanhydrid in einem Gemisch aus 6,2 l MTBE und 2,2 l Propylencarbonat bereitet. 5 ml dieser Lösung wurden zu jeweils einem 10 mg freier Lipase entsprechenden Aliquot der immobiliserten Lipase gegeben. Es wurde 12 h bei RT geschüttelt, von der immobilisierten Lipase abfiltriert und der Umsatz und die Enantioselektivität des nicht-a-cylierten Antipoden per GC bestimmt und in Tabelle 3 aufgelistet. Die Filterrückstände wurden mit MTBE gewaschen und erneut eingesetzt, um die Standzeit zu bestimmen. Die Versuchsdurchführung wurde bis zu 10 mal wiederholt. Als immobilisierte Lipasen wurden die nach Beispiel 1.2.2 d) hergestellten Lipasen verwendet. Zum Vergleich wurde der analoge Versuch mit der freien Lipase durchgeführt.

**Tabelle 4.1**

| | | |
|---|---|---|
| Umsatz (U in [%]) und Enantioselektivität (ee in [%]) des nich-t-acylierten Antipoden bei der enantioselektiven Acylierung von racemischen trans-2-Methoxycyclohexanol in Gegenwart der freien Lipase in einem diskontinuierlichen Verfahren. Das Verfahren wurde mit der aus dem vorhergehenden Versuch isolierten Lipase bis zu 10 mal wiederholt. | | |

| freie Lipase | | |
|---|---|---|
| Lauf | U | ee |
| 1 | 54,5 | 99 |
| 2 | 47,3 | 88 |
| 3 | 43,2 | 75 |
| 4 | 29,4 | - |
| 5 | 14,5 | - |
| 10 | 3,4 | - |

**Tabelle 4.2**

| | | |
|---|---|---|
| Umsatz (U in [%]) und Enantioselektivität (ee in [%]) des nich-t-acylierten Antipoden bei der enantioselektiven Acylierung von racemischen trans-2-Methoxycyclohexanol in Gegenwart der jeweiligen immobilisierten Lipase in einem diskontinuierlichen Verfahren. Das Verfahren wurde mit den aus dem vorhergehenden Versuch isolierten immobilisierten Lipase bis zu 10 mal wiederholt. | | |

| immobilisierte Lipase Pseudomonas burkholderi (Bsp. 1.2.2 d)) | | |
|---|---|---|
| Lauf | U | ee |
| 1 | 55,0 | > 99 |
| 2 | 54,5 | > 99 |
| 3 | 53,8 | > 99 |
| 4 | 54,2 | > 99 |
| 5 | 54,6 | > 99 |
| 10 | 54,6 | > 99 |

### Beispiel 4.2

### Kontinuierliches Verfahren

In eine Chromatographiesäule mit 1 cm Durchmesser wurde die immobilisierte Lipase aus Beispiel 1.2.2. f) eingefüllt, und es wurde mittels einer Pumpe ein auf 30-35 ml/h eingestellter Strom der Eduktlösung über das Immobilisat gepumpt. In regelmäßigen Abständen wurden Proben genommen, per GC der Umsatz und die Enantioselektivität bestimmt und in Tabelle 4.4 aufgelistet. Zum Vergleich wurde ein Lyophilisat der freien Lipase eingesetzt.

**Tabelle 4.3**

| | | | | | | | | | | | | |
|---|---|---|---|---|---|---|---|---|---|---|---|---|
| Umsatz (U) und Enantioselektivität (ee) in [%], gemessen in regelmäßigen Zeitabständen bei einem kontinuierlichen Verfahren der enantioselektiven Acylierung von racemischen trans-2-Methoxycyclohexanol. Die Werte der immobilisierte Lipase aus Beispiel 1.2.2 d) werden mit den Werten der freien Lipase verglichen. | | | | | | | | | | | | |

| Zeit | 10 h | | 20 h | | 50 h | | 100 h | | 150 h | | 200 h | |
|---|---|---|---|---|---|---|---|---|---|---|---|---|
| | U | ee | U | ee | U | ee | U | ee | U | ee | U | ee |
| freie Lipase | 54,9 | 99 | 42,3 | 69 | 28,2 | 28 | 19,4 | - | - | - | - | - |
| Imm. Lipase Beisp.1.2.2.f) | 55,0 | 99 | 53,8 | 99 | 54,6 | 99 | 54,0 | 99 | 54,2 | 99 | 53,9 | 99 |

## Patentansprüche

1. Verfahren zur Herstellung von immobilisierter Lipase, indem man eine Rohlipaselösung mit Polyolefin-Partikel in Kontakt bringt.

2. Verfahren nach Anspruch 1, dadurch gekennzeichnet, daß man als Rohlipaselösung eine zellfreie Kulturbrühe verwendet, die durch
a) Kultivieren eines Lipase-produzierenden Organismus,
b) gegebenenfalls anschließendem Dispergieren und/oder Homogenisieren des Organismus in einer Lösung und
c) anschließendem Abtrennen der Zellen
erhältlich ist.

3. Verfahren nach Anspruch 1 oder 2, dadurch gekennzeichnet, daß man als Lipase die Lipase aus Pseudomonas burkholderia oder Pseudomonas aeruginosa oder als Lipase-produzierenden Organismus Pseudomonas burkholderia oder Pseudomonas aeruginosa verwendet.

4. Immobilisierte Lipase, erhältlich nach einem Verfahren gemäß Anspruch 1.

5. Verfahren zur enzymkatalytischen Umwandlung oder enantioselektiven Umwandlung von Substraten, dadurch gekennzeichnet, daß man die Substrate in Gegenwart der immobilisierten Lipase gemäß Anspruch 4 umsetzt.

6. Verfahren nach Anspruch 5, dadurch gekennzeichnet, daß man als Substrate Alkohole, Amine oder Aminosäureester verwendet und diese in Gegenwart eines Acylierungsmittels acyliert oder enantioselektiv acyliert.

7. Verfahren nach Anspruch 5, dadurch gekennzeichnet, daß man als Substrate Carbonsäureester verwendet und diese verseift oder enantioselektiv verseift.

8. Verfahren zur Herstellung optisch aktiver Verbindungen, dadurch gekennzeichnet, daß man Stereoisomerengemische oder Racemate von Subtraten, die sich enzymkatalytisch von Lipasen umsetzen lassen, enantioselektiv in Gegenwart der immobilisierten Lipase nach Anspruch 4 umsetzt und anschließend die Gemische auftrennt.

9. Verfahren nach Anspruch 8, dadurch gekennzeichnet, daß man als Substrate Alkohole, Amine oder Aminosäureester verwendet und diese in Gegenwart eines Acylierungsmittels acyliert oder enantioselektiv acyliert.

10. Verfahren nach Anspruch 8, dadurch gekennzeichnet, daß man als Substrate Carbonsäureester verwendet und diese verseift oder enantioselektiv verseift.

11. Verwendung der immobilisierten Lipase gemäß Anspruch 4 als Katalysator in chemischen Reaktionen.
